# EUROPEAN PATENT APPLICATION

(11) **EP 3 437 687 A1**
(43) Date of publication of application: **06.02.2019**
(21) Application number: 17788961.5
(22) Date of filing: 18.01.2017
(51) Int. Cl.: A61M 21/02, A01K 15/00, A61B 5/0245, G09B 19/00

(54) **ASSISTANCE METHOD, ASSISTANCE SYSTEM, AND PROGRAM**

(30) Priority: 27.04.2016 JP 2016089915
(71) Applicant: Unicharm Corporation, Ehime 799-0111 (JP)
(72) Inventor: GOUDA, Ayami, Kanonji-shi Kagawa 769-1602 (JP)
(74) Representative: Dolleymores
(86) International application number: PCT/JP2017/001511
(87) International publication number: WO 2017/187671

(57) **Abstract**

There is provided a method for enhancing heart rate variability of an infant or an animal of a low age or a low age in month. An assist method for assisting to enhance a function of an automatic nervous system of a dependent or an animal cared by a user, comprises, by a computer: a step of monitoring a first index configured to indicate a function of an automatic nervous system of the user; a step of monitoring a second index configured to indicate the function of the automatic nervous system of the dependent or the animal; and a step of providing training contents to the user, wherein the training contents are configured to enhance the function of the automatic nervous system of the user determined based on at least the second index.

## Description

### [TECHNICAL FIELD]

The present invention relates to an assist method and an assist system which assist to enhance a function of an automatic nervous system of a dependent or an animal cared by a user.

### [BACKGROUND ART]

In recent years, various child care assist apparatuses and child care assist methods are proposed. Patent Literature 1 discloses a child care assist apparatus and a child care assist method which assist acquisition of signs for an infant. According to this method, a sign such as a body gesture or a hand gesture is associated with a specific word to make an infant to learn by heart, so that it is possible to encourage communication with the infant and assist child caring.

### [CITATION LIST]

### [PATENT LITERATURE]

Patent Literature 1: Japanese Patent Application Laid-Open No. 2015-176436

### [SUMMARY OF INVENTION]

By the way, in recent years, a heart rate variability biofeedback (HRV-BF) method is gaining attention for improvement of depression, an uneasy state and a sleep disorder. The heart rate variability biofeedback method includes controlling breathing to enhance heart rate variability (HRV) which is an index indicating an automatic nervous system activity. In addition, the heart rate variability is defined by fluctuation of a heart beat rhythm. By enhancing the heart rate variability according to the heart rate variability biofeedback method, it is possible to enhance the automatic nervous system activity.

However, dependents such as children, old people, patients and handicapped people or animals have difficulty in carrying out the heart rate variability biofeedback method by themselves. It is therefore desired to provide a method for enhancing a function of an automatic nervous system of a dependent or an animal, and/or a method for assisting to enhance the function.

An assist method according to one aspect relates to an assist method for assisting to enhance a function of an automatic nervous system of a dependent or an animal cared by a user. The assist method comprises, by a computer: a step of monitoring a first index configured to indicate a function of an automatic nervous system of the user; a step of monitoring a second index configured to indicate the function of the automatic nervous system of the dependent or the animal; and a step of providing training contents to the user, wherein the training contents are configured to enhance the function of the automatic nervous system of the user determined based on at least the second index.

### [BRIEF DESCRIPTION OF DRAWINGS]

Fig. 1 is a schematic configuration diagram of a communication system including an assist system.
Fig. 2 is a block diagram of the assist system.
Fig. 3 is a view illustrating a flow related to an operation of the assist system.
Fig. 4 is a view illustrating a schematic example of a power spectrum of heart rate variability.
Fig. 5 is a view illustrating an LF component value related to a heart rate variability property of a parent.
Fig. 6 is a view illustrating an LF component value related to a heart rate variability property of a child.

### [DESCRIPTION OF EMBODIMENTS]

At least following matters will be become apparent from disclosure of the description and the accompanying drawings.

An assist method for assisting to enhance a function of an automatic nervous system of a dependent or an animal cared by a user, comprising, by a computer:
a step of monitoring a first index configured to indicate a function of an automatic nervous system of the user;
a step of monitoring a second index configured to indicate the function of the automatic nervous system of the dependent or the animal; and
a step of providing training contents to the user, wherein the training contents are configured to enhance the function of the automatic nervous system of the user determined based on at least the second index.

The inventors of the present invention have found a phenomenon that the function of the automatic nervous system of the dependent or the animal synchronizes with the function of the automatic nervous system of the user. More specifically, the inventors have demonstrated that heart rate variability of a child synchronizes with heart rate variability of a parent of this child. A synchronization phenomenon of the function of the automatic nervous system such as the heart rate variability is expected be realized between the dependent or the animal and the caregiver, too. Accordingly, the inventors of the present invention have obtained knowledge that the function of the automatic nervous system of the dependent or the animal which has difficulty in carrying out a biofeedback method by itself can be enhanced by enhancing the function of the automatic nervous system of the user who cares the dependent or the animal.

To enhance the function of the automatic nervous system of the dependent, the dependent preferably carries out the biofeedback method. However, the dependents such as children, old people, patients or handicapped people have difficulty in carrying out the biofeedback method by themselves. The inventors have considered enhancing the function of the automatic nervous system of the dependent by enhancing the function of the automatic nervous system of the user according to the synchronization phenomenon of the function of the automatic nervous system such as heart rate variability.

Furthermore, according to the method, a training is determined for the user who can carry out the training based on a state (second index) of the function of the automatic nervous system of the dependent or the animal who has difficulty in carrying out the training by itself. Thus, the state (second index) of the function of the automatic nervous system of the dependent or the animal is reflected in the training. When the user carries out the training, it can be expected that the function of the automatic nervous system of the dependent or the animal can be enhanced together with the function of the user.

According to one preferable aspect, the training contents are determined based on both the first index and the second index. The training contents are determined by taking into account not only the state (second index) of the function of the automatic nervous system of the dependent or the animal but also the state (first index) of the function of the automatic nervous system of the user. Consequently, it can be expected that the function of the automatic nervous system of the dependent or the animal can be more effectively enhanced together with the function of the user.

According to one preferable aspect, the first index and the second index are calculated based on heart rate variability.

According to one preferable aspect, the assist method comprises: a step of providing to the user an image, a video image, a voice or vibration configured to assist the training contents provided to the user; and a step of providing a training result related to a second index configured to indicate at least the function of the automatic nervous system of the dependent or the animal after the image, the video image, the voice or the vibration for assisting the training contents ends.

By making the user confirm the enhanced function of the automatic nervous system of the dependent or the animal, the user can confirm that the function of the automatic nervous system of the dependent or the animal is enhanced by the training carried out by the user. Consequently, it is possible to highly satisfy the user or motivate the user to continue the training.

According to one preferable aspect, the training result includes data configured to indicate a correlation between the function of the automatic nervous system of the user and the function of the automatic nervous system of the dependent or the animal after the training.

The user can confirm synchronization of the function of the automatic nervous system of the user and the function of the automatic nervous system of the dependent and the animal. Consequently, it is possible to improve user's motivation to carry out the training.

According to one preferable aspect, the assist method further comprises a step of providing a notification configured to encourage the user to contact the dependent or the animal before the image, the video image, the voice or the vibration configured to assist the training contents starts.

By broadcasting a notification of a method for synchronizing the function of the automatic nervous system of the user and the function of the automatic nervous system of the dependent or the animal (such a method is contacting with the dependent or the animal), it is possible to reliably synchronize an effect of the training carried out by the user with the dependent or the animal. Furthermore, the user can confirm a change in the function of the automatic nervous system of the dependent or the animal by a contact action of the user, and consequently recognize the effect of the contact action.

According to one preferable aspect, the assist method further comprises a step of causing the user to input a questionnaire related to an effect of the training before start of or after the end of the image, the video image, the voice or the vibration configured to assist the training contents, and the training contents are determined based on at least a result of the questionnaire inputted by the user and the second index.

By feeding back the training effect by means of the questionnaire, it is possible to determine effective training contents per user.

According to one preferable aspect, the assist method further comprises a step of notifying the user of a timing to start the training.

By notifying the user of the timing to start the training, it is possible to encourage the user to start the training at an appropriate timing.

According to one preferable aspect, the start of the training is notified based on an activity amount of the dependent or the animal in a predetermined period.

By deciding necessity of the training based on the activity amount of the dependent or the animal in the predetermined period, it is possible to encourage the user to start the training at a more appropriate timing.

Furthermore, the training is preferably notified based on not only the activity amount of the dependent or the animal in the predetermined period but also user information. The user information is information such as an activity amount of the user in the predetermined period, a transition of the first index indicating the function of the automatic nervous system in the predetermined period, or a cumulative value of the first indices.

According to one preferable aspect, the assist method further includes a step of calculating a target value of the second index configured to indicate the function of the automatic nervous system of the dependent based on at least one element of a weight at a time of birth, a sex, an age in day and a period from pregnancy to birth of the dependent, and the training contents to be provided to the user are determined based on a difference between the target value and the second index.

A reference value of the second index indicating the function of the automatic nervous system of the dependent (e.g., infant) is known to differ based on the weight at the time of birth, the sex, the age in day and the period from pregnancy to birth. By determining the target value based on at least one element of the weight at the time of birth, the sex, the age in day and the period from pregnancy to birth, it is possible to determine more appropriate training contents.

The present invention includes a program which causes the computer to execute the assist method, and the assist system which executes the assist method, too, in view of disclosure of the description and the accompanying drawings.

An assist system and an assist method according to one embodiment will be described below with reference to the drawings. In addition, the same or similar portions will be assigned the same or similar reference symbols in the following drawings. In this regard, it should be born in mind that the drawings are schematic, and a ratio of each dimension is different from an actual one. Therefore, each specific dimension needs to be decided in view of the following description. Furthermore, some of respective dimension relationships and ratios are different between the mutual drawings.

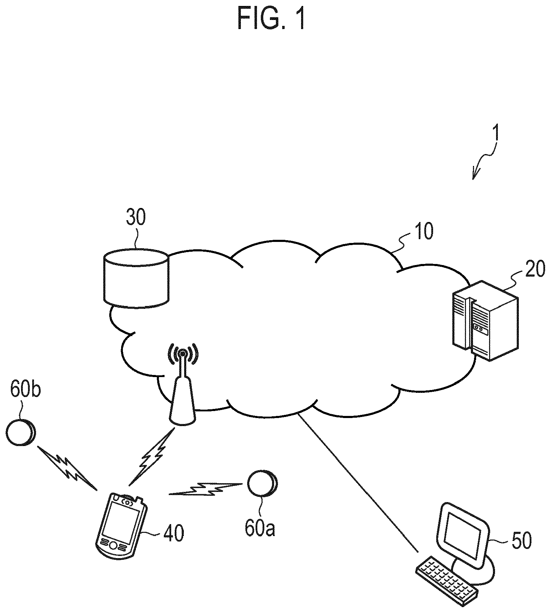

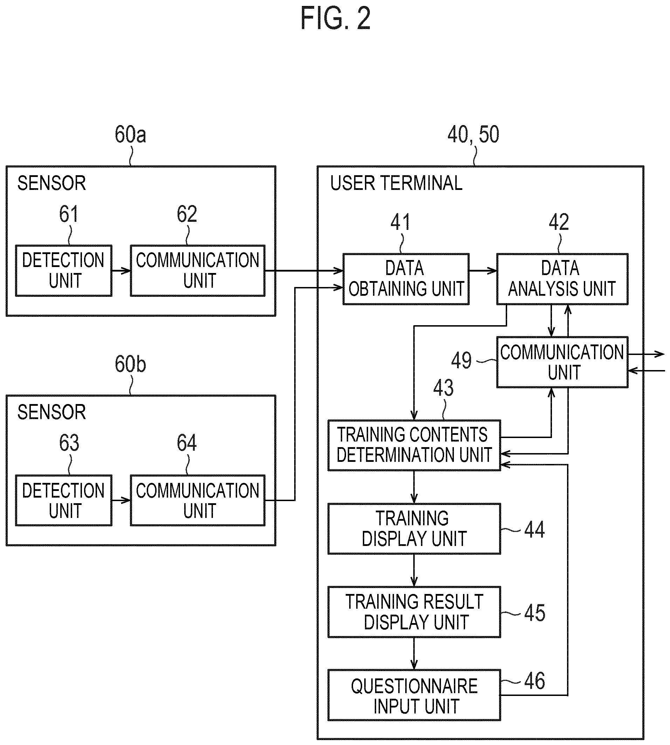

Fig. 1 is an entire schematic configuration diagram of an assist system 1 according to the present embodiment. Fig. 2 is a block diagram of the assist system 1. In the present embodiment, the assist system 1 is a system which provides an assist method for assisting to enhance a function of an automatic nervous system of a child of a user and, more particularly, an infant.

The assist system 1 includes a communication network 10, a server 20 on a network, a database 30, user terminals 40 and 50 and sensors 60a and 60b. The communication network 10 is a wired or wireless communication network which connects to the server 20 on the network, the database 30 and the user terminal 40 and 50 so as to be able to communicate with each other. In addition, the communication network 10 may include the Internet.

The database 30 is a database which stores various pieces of necessary information such as information of the user who uses the assist system 1 and a child of the user for the assist system 1. The database 30 may store information (so-called big data) related to multiple users and children of these users.

The sensor 60a includes a detection unit 61 which detects a heart rate of the user. The sensor 60b includes a detection unit 63 which detects a heart rate of a child (an infant in particular) of the user. The sensors 60a and 60b may be configured to be attachable to the user or the child. Furthermore, the sensors 60a and 60b may not be attached to the user or the child, and be able to detect a heart rate without contacting the user or the child. The sensors 60a and 60b include communication units 62 and 64 which transmit heart rate data measured by the detection units 61 and 63 to data obtaining units 41 of the user terminal 40 and 50, respectively.

In Fig. 1, the two sensors 60a and 60b are provided for the user and the child. However, the sensor may be shared between the user and the child. In this case, it is necessary to measure a user's heart rate and a child's heart rate alternately at a necessary timing.

The user terminal 40 is a mobile communication terminal such as a smartphone or a mobile telephone terminal. The user terminal 50 is a personal computer. Fig. 1 illustrates the two terminals 40 and 50 as the user terminals. However, there may be one user terminal.

By downloading an application for executing the assist method from the server 20 on the network, the user can execute the assist method by using the user terminal 40 or 50.

The user terminal 40 or 50 may include the data obtaining unit 41, a data analysis unit 42, a training contents determination unit 43, a training display unit 44, a training result display unit 45, a questionnaire input unit 46 and a communication unit 49. The communication unit 49 can communicate with the server 20 on the network.

The data obtaining unit 41 obtains heart rate data measured by the sensors 60a and 60b. The data analysis unit 42 calculates an index indicating a function of an automatic nervous system from the heart rate data. More specifically, the data analysis unit 42 first calculates data related to a time interval of beats from the heart rate data. The heart rate variability indicates temporary variability (heart rate fluctuation) of the time interval of the beats.

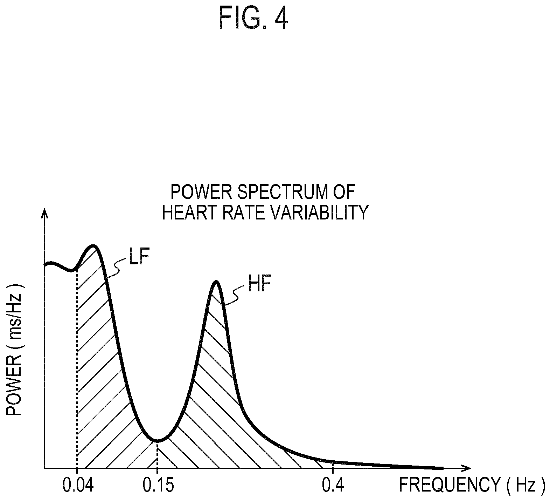

Generally, the time interval of the beats fluctuates over time. The data analysis unit 42 further calculates a power spectrum of heart rate variability data. The power spectrum is data indicating frequency dependency of the heart rate variability data (see Fig. 4, too).

In addition, a high frequency component such as a component of 0.15 Hz to 0.4 Hz in the power spectrum of the heart rate variability data is referred to as an HF component. A low frequency component such as a component of 0.04 Hz to 0.15 Hz is referred to as an LF component. These HF component and LF component are indices indicating the degrees of activity of the automatic nervous system. More specifically, a cumulative value (HF component value) of the components of 0.15 Hz to 0.4 Hz, and a cumulative value (LF component value) of components of 0.04 Hz to 0.15 Hz can be used as the indices.

When, for example, a parasympathetic nerve is dominant, the HF component appears, and therefore the HF component value may be defined as the degree of activity of the parasympathetic nerve. Furthermore, even when a sympathetic nerve is dominant or the parasympathetic nerve is dominant, the LF component value appears, and therefore a ratio of the LF component and the HF component may be defined as the degree of activity of the sympathetic nerve.

The training contents determination unit 43 provides, to the user, training contents for enhancing the function of the automatic nervous system of the user. The training contents determination unit 43 provides at least one preferable training contents from a plurality of items of stored training contents to the user.

The training contents determination unit 43 provides to the user the training contents for enhancing the function of the automatic nervous system of the user based on at least the second index (HF component and/or the LF component) indicating the function of the automatic nervous system of the child. The training contents which the user needs to carry out are determined based on at least the second index indicating the function of the automatic nervous system of the child. Consequently, it is possible to make the user carry out an appropriate training for enhancing the automatic nervous system of the child.

Instead, the training contents determination unit 43 may provide to the user the training contents for enhancing the function of the automatic nervous system of the user based on both of the first index (HF component and/or the LF component) indicating the function of the automatic nervous system of the user and the second index (HF component and/or the LF component) indicating the function of the automatic nervous system of the child. As described above, the function of the automatic nervous system of the child synchronizes with the function of the automatic nervous system of the user (parent). Consequently, by determining training contents by taking into account a state (first index) of the function of the automatic nervous system of the user, it can be expected that the function of the automatic nervous system of the child can be more effectively enhanced together with the function of the user.

Training contents may be, for example, deep breathing at a predetermined rhythm. That is, the training contents may be an action of repeatedly inhaling breath for a predetermined time and then exhaling the breath for a predetermined time a predetermined number of times. Furthermore, the training contents may be an action of patting a child's body at a predetermined rhythm. Furthermore, the training contents may be an action of listening to a sound at a predetermined rhythm or shaking a body at a predetermined rhythm.

More specifically, the training contents determination unit 43 specifies a combination of a training type and a cycle of the above predetermined rhythm from the above examples, and provides a training to the user. In a case of, for example, deep breathing, the training contents determination unit 43 provides a combination of an inhaling time of the user, an exhaling time and the number of times of repetition to the user.

In a more specific example, training contents can be determined according to a difference between the second index (calculated from actually measured data) indicating the function of the automatic nervous system of the child and a target value indicating the function of the automatic nervous system of the child. The target value may be determined based on, for example, the age of the child of the user. More specifically, the database 30 stores an average value of the second indices indicating functions of automatic nervous systems of general children. The training contents determination unit 43 obtains as the target value the average value of the second indices of the children at the same age as the age of the child from the database 30 via the communication unit 49 and the server 20. The training contents determination unit 43 can determine the training contents based on the difference between this target value and the second index indicating the function of the automatic nervous system of the child.

Furthermore, the above target value may be calculated based on at least one element of the weight at the time of birth, the sex, the age in day and the period from pregnancy to birth of the child. A heart rate variability property indicating a function of an automatic nervous system changes according to the weight at the time of birth, the sex, the age in day and the period from pregnancy to birth of the child. Consequently, by setting the target value based on these indices, it is possible to determine more appropriate training contents for the user and the child of the user. Furthermore, the above target value is preferably determined by taking into account a result of the second index indicating the function of the automatic nervous system of the child measured before a training starts, and a result of a questionnaire described below and carried out before the training starts.

The training display unit 44 provides to the user terminals 40 and 50 an image, a video image, a voice or vibration for assisting training contents started by the user. For example, the user terminals 40 and 50 may output a voice saying "inhale" at a timing to inhale, and output a voice saying "exhale" at a timing to exhale. Furthermore, the training display unit 44 may display on the user terminals 40 and 50 a video image which enables perceptual recognition of a timing to inhale and a timing to exhale. Furthermore, the training display unit 44 may change a vibration pattern of the vibration between the timing to inhale and the timing to exhale. When the vibration is used, people having difficulty in eyes and ears can carry out trainings. Furthermore, users can carry out more appropriate trainings based on tactile senses even at places with noisy surroundings.

The training result display unit 45 provides a training result to the user terminals 40 and 50 after the training ends, i.e., after the image, the video image, the voice or the vibration for assisting the training contents ends.

The training result shows information related to at least the second index indicating the function of the automatic nervous system of the child during the training and/or after the training. Furthermore, the training result may show data related to both the first index indicating the function of the automatic nervous system of the user and the second index indicating the function of the automatic nervous system of the child.

Furthermore, the training result preferably includes data indicating a correlation between the function of the automatic nervous system of the user and the function of the automatic nervous system of the child. This correlation may be information related to a difference between the HF component value of the heart rate variability of the user and the HF component value of the heart rate variability of the child, and/or a difference between the LF component value of the heart rate variability of the user and the LF component value of the heart rate variability of the child. Furthermore, the correlation may be information related to a correlation coefficient of the heart rate variability of the user and the heart rate variability of the child. Consequently, the user can confirm synchronization of the function of the automatic nervous system of the user and the function of the automatic nervous system of the child, and improve user's motivation to carry out a training.

Furthermore, the training result display unit 45 may provide a transition of past training results to the user terminals 40 and 50. Consequently, the user can confirm the transition of the training effect.

The questionnaire input unit 46 displays on the user terminals 40 and 50 the questionnaire related to the training effect before the start of or after the end of the image, the video image, the voice or the vibration for assisting the training contents, and makes the user input the answer. It is possible to calculate the correlation between the training contents and the training effect based on a result of the questionnaire inputted by the user. By calculating the correlation between the training contents and the training effect, it is possible to determine which training is effective for each user and child.

The questionnaire result inputted from the questionnaire input unit 46 is preferably fed back to the training contents determination unit 43. The training contents determination unit 43 can determine more appropriate training contents per user and child of the user based on the fed-back questionnaire result. That is, after the user inputs the questionnaire, the training contents determination unit 43 preferably determines the training contents based on at least the questionnaire result inputted by the user and the above second index.

As questionnaire contents, a POMS which is known as an emotion measurement index can be used. By answering question items such as "mind is clear", "tired" and "cannot concentrate" at five levels, it is possible to convert a questionnaire result into numeric values. The training contents determination unit 43 may preferentially select training contents of a high evaluation based on the questionnaire result converted into numerical values.

Furthermore, well-known quick inventory of depressive symptomatology (QIDS-J) may be used as questionnaire contents.

The questionnaire contents preferably include both of matters related to the user and matters related to the child.

More specific training contents, a result obtained by this training, a time zone during which the training is carried out, information of the user who has carried out this training and information of the child of the user are stored in the database 30 via the communication unit 49 by way of the server 20. The database 30 preferably stores the above information of multiple users.

The user information includes the age, the sex and the weight of the user. The child information includes the age (more preferably, the age in month), the sex and the weight of the child. Furthermore, the user and child information may include values of the first index and the second index indicating the functions of the automatic nervous systems of the user and the child.

When multiple users store results of trainings carried out by the users in the database 30, it is possible to specify which training is particularly effective to synchronize functions of automatic nerves. That is, by feeding back information related to the multiple users, the training contents determination unit 43 can provide more appropriate trainings to the users.

More specifically, the training contents determination unit 43 requests the server 20 via the communication unit 49 to obtain training contents which are regarded as effective in a population, from the population including the same or similar elements as or to elements such the age, the sex and the weight of the user, and a current value of the second index, the age (more preferably, the age in month), the sex and the weight of the child.

The server 20 can select the training contents which are regarded as effective in the population from the database 30, and transmit the effective training contents to the user terminals. Consequently, the training contents determination unit 43 can determine training contents by taking into account training results of users having the same or similar individual information, too, and provide more effective training contents to the users.

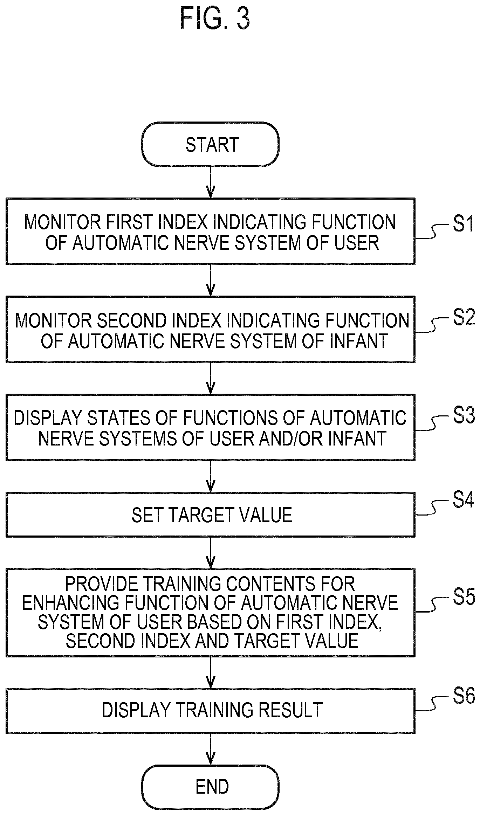

Next, an operation of the assist system will be described. Fig. 3 is a view illustrating a flow related to the operation of the assist system.

First, when the user starts the assist system, the sensors 60a and 60b monitor heart rates of the user and the child, respectively (steps S1 and S2). Any one of the user's heart rate and the child's heart rate may be measured first. In addition, the sensors 60a and 60b may monitor the heart rates at all times. The data analysis unit 42 calculates the power spectrum of heart rate variability from heart rate measurement results, and calculates the HF component value and the LF component value (first index) of the user and the HF component value and the LF component value (second index) of the child.

In addition, the assist system 1 may be configured to be able to automatically broadcast to the user a notification for encouraging the user to carry out a training described below. For example, the assist system broadcasts to the user a notification for encouraging the user to carry out a training at a date set by the user in advance. The notification to the user may be broadcasted by a sound emitted from the user terminals 40 and 50 or vibration of the user terminals 40 and 50.

Furthermore, start of the training may be notified based on an activity amount of the child in a predetermined period (e.g., one day). The activity amount of the child can be measured by using a known activity monitor. The activity amount of the child can be also measured by, for example, an acceleration sensor. The acceleration sensor monitors a detection value, so that it is possible to estimate the activity amount of the child in the predetermined period. When the activity amount of the child in the predetermined period is smaller than a predetermined threshold, the start of the training may be notified to the user. The start of the training may be notified based on not only the activity amount of the child in the predetermined period but also information such as the transition of the second index indicating the function of the automatic nervous system of the child in the predetermined period (e.g., one day) or the cumulative value of the second indices. Furthermore, the start of the training is preferably notified based on user information, too. The information of the user is information such as an activity amount of the user in the predetermined period (e.g., one day), a transition of the first index indicating the function of the automatic nervous system in the predetermined period (e.g., one day), or a cumulative value of the first indices.

Next, data described below and indicating the state of the function of the automatic nervous system of the child before the training starts is optionally displayed on the user terminals 40 and 50 (step S3). The state of the function of the automatic nervous system can be displayed at, for example, a plurality of levels. For example, the measured state of the function of the automatic nervous system of the child can be classified into three levels of a relatively high level (the state is sufficiently higher than the target value), a middle level (the state is at the target value or takes a value close to the target value) or a relatively low level (the state is sufficiently lower than the target value). The assist system 1 displays at which level of the three levels the current state of the child is, on the user terminals 40 and 50.

Next, a step of inputting an object for calming the child or making the child active is optionally performed. In a case of the object for calming the child, a training for enhancing an activity of a parasympathetic nerve is necessary. Furthermore, in a case of the object for making the child active, a training for enhancing an activity of a sympathetic nerve is necessary. Such selection of the object is preferably reflected in the training contents determination unit 43 described below.

Next, the target value of the function of the automatic nervous system of the child is set (step S4). The target value can be automatically set from the average value of the second indices indicating functions of automatic nervous systems of a child group satisfying the same condition as the child of the user.

After the target value is set, the training contents determination unit 43 provides to the user the training contents for enhancing the function of the automatic nervous system of the user based on at least the second index (HF component and the LF component) indicating the function of the automatic nervous system of the child (step S5). As described above, the training contents are preferably determined based on both the second index indicating the function of the automatic nervous system of the child and the first index indicating the function of the automatic nervous system of the user. Furthermore, the training contents may be determined by taking into account the result of the questionnaire inputted by the user in the past.

The user selects whether or not it is necessary to carry out the training provided by the assist system 1. When the user selects to start the training, an image, a video image, a voice or vibration for assisting the training contents is provided to the user. The user can carry out the training in combination with the image, the video image, the voice or the vibration for assisting the training contents.

Before the image, the video image, the sound or the vibration for assisting the training contents starts, a notification for encouraging the user to contact the child is preferably broadcasted. For example, before the training starts, a voice such as "hold hands", "hold in your arms" or "lie down together", or an image or a video image (e.g., a moving image illustrating that the child is held) for encouraging perceptual recognition of these actions is preferably outputted to the user terminals 40 and 50.

Furthermore, notification contents for encouraging the user to contact the child may be automatically determined by a computer according to the age or the age in month of the child. For example, a training of the user is effective for an infant of a relatively high age in month when the infant is trying to sleep or sleeping. Hence, in a case of the infant of a relatively high age in month, a voice such as "lie down together" is preferably notified. Thus, an appropriate notification is preferably broadcasted according to the age of the child (dependent).

After the image, the video image, the voice or the vibration for assisting the training contents ends, the assist system 1 displays on the user terminals the training result related to the first index indicating the function of the automatic nervous system of the user and the second index indicating the function of the automatic nervous system of the child (step S6).

Preferably, after the image, the video image, the voice or the vibration for assisting the training contents ends, the assist system 1 makes the user input the questionnaire related to the training effect.

Furthermore, before the image, the video image, the voice or the vibration for assisting the training contents starts, the assist system 1 may make the user input a questionnaire related to current states of bodies and minds of the user and/or the child. Questionnaire contents may be the same as contents related to the above training result.

In addition, the program which causes the computer and, more particularly, the user terminal to execute the above method is also included in the scope of the present invention. The program may be stored in a recording medium. By using this recording medium, the above program can be installed in the user terminal, for example. In this regard, the recording medium having the above program stored thereon may be a non-transitory recording medium. The non-transitory recording medium is not limited in particular, and may be a recording medium such as a CD-ROM.

### (Example)

16 pairs of mothers and children carried out a training, and effects of the training were measured. The ages in month of the children were three to five months. "MyBeat (UNION TOOL. CO.)" was attached as sensors which measure heart rates to breasts of the mothers and the children.

First, the children were laid down on futon and were kept in a rest state for five minutes in a state where the mothers sat (a pre-rest state). Then, the mothers sat on chairs, and breathed deeply for 15 minutes (inhaled for four seconds and exhaled for six seconds) embracing the children vertically (during a training). Subsequently, the children were laid down on futon again, and kept in the rest state for five minutes in a state where the mothers sat (a post-rest state). The heart rates of the mothers and the children were measured from the pre-rest state to the post-rest state, and LF component values were calculated by power spectrum analysis.

The measured heart rates were frequency-analyzed (power spectrum analysis) by "WHS-1 advanced viewer software (WIN Frontier Co,. Ltd.)", and an HF component (a cumulative value of 0.15 Hz to 0.4 Hz) and an LF component (a cumulative value of 0.04 Hz to 0.15 Hz) were calculated.

Similarly, the same action as the above training was performed by spontaneous breathing instead of deep breathing in another day's schedule to measure heart rates of the mothers and the children from the pre-rest state to the post-rest state and calculate the LF component value by the power spectrum analysis.

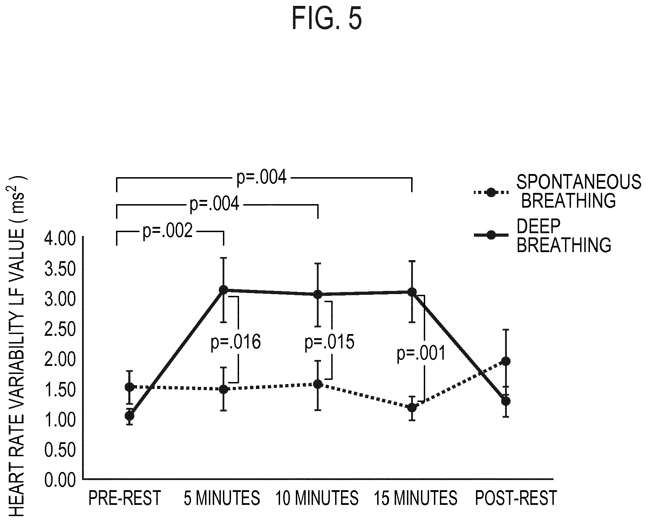

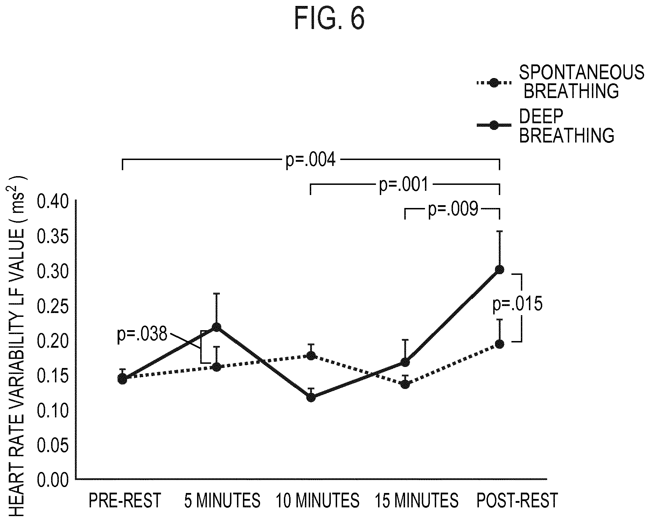

Fig. 5 illustrates a graph illustrating a transition of the LF component values of the mothers during the training. Fig. 6 illustrates a graph illustrating a transition of the LF component values of the children during the training.

FIG. 5 illustrates that the LF component values of the mothers during deep breathing significantly became higher than in the post-rest state and the post-rest state. Furthermore, FIG. 6 illustrates that the LF component values of the children significantly rose in the post-rest state after the training. As a result of the training which uses deep breathing in particular, the LF component values of the children significantly became high.

The above result shows that the functions (heart rate variability) of the automatic nervous systems of the children and the functions (heart rate variability) of the automatic nervous systems of the mothers synchronize. This means that the functions of the children who cannot carry out the biofeedback method by themselves can be enhanced by enhancing the functions of automatic nervous systems of the mothers.

Furthermore, the inventors of the present invention found that a synchronization phenomenon of the functions (heart rate variability) of the automatic nervous systems significantly appeared in infants of low ages in month such as three to five months in particular. Hence, the above assist system and assist method are preferably used by the infants and parents of the infants.

Furthermore, the above assist system and assist method can be also used by mothers and unborn babies. In this case, too, it can be expected that automatic nerves of the unborn babies are enhanced. In this case, heart rates of the unborn babies may be measured by non-contact type sensors.

As described above, contents of the present invention have been disclosed in the embodiment of the present invention. However, it should not be understood that the description and the drawings which are part of this disclosure limit the present invention. This disclosure makes various alternative embodiments, examples and application techniques obvious for one of ordinary skill in the art.

The above embodiment has described that a training is performed by using the power spectrum analysis of heart rates of parents and children. However, brain waves can be also used instead of the heart rates. The synchronization phenomenon of the brain waves of parents and children can be expected similar to the heart rates. Consequently, there can be provided an assist system and an assist method which assist effective trainings.

Furthermore, the above assist system and assist method are not limited to those which are carried out by parents and children of the parents. For example, the assist system and assist method can be also carried out by pets and owners of the pets. That is, functions of automatic nervous systems can synchronize between a user and an animal (pet) cared by the user, too, so that the above assist system and assist method may be applied to the user and the pet.

Furthermore, a sensor which measures a heart rate may be provided to the user terminal 40. For example, the sensor which measures the heart rate can be configured as a camera and an image analysis unit provided to the user terminal 40. By performing image analysis on data obtained by capturing images of fingers of a user and a child pressed against the camera, it is possible to measure heart rates of the user and the child.

This application claims priority to Japanese Patent Application No. 2016-89915 filed on April 27, 2016, the entire contents of which are incorporated by reference herein.

### [INDUSTRIAL APPLICABILITY]

According to the present invention, it is possible to provide to a user a method for enhancing a function of an automatic nervous system of a dependent or an animal, and/or a method for assisting to enhance the function.

### [REFERENCE SIGNS LIST]

40, 50 USER TERMINAL
60a, 60b SENSOR

## Claims

1. An assist method for assisting to enhance a function of an automatic nervous system of a dependent or an animal cared by a user, comprising, by a computer:
a step of monitoring a first index configured to indicate a function of an automatic nervous system of the user;
a step of monitoring a second index configured to indicate the function of the automatic nervous system of the dependent or the animal; and
a step of providing training contents to the user, the training contents being configured to enhance the function of the automatic nervous system of the user determined based on at least the second index.

2. The assist method according to claim 1, wherein the training contents are determined based on both the first index and the second index.

3. The assist method according to claim 1 or 2, wherein the first index and the second index are calculated based on heart rate variability.

4. The assist method according to any one of claims 1 to 3, further comprising:
a step of providing to the user an image, a video image, a voice or vibration configured to assist the training contents provided to the user; and
a step of providing a training result related to a second index configured to indicate at least the function of the automatic nervous system of the dependent or the animal after the image, the video image, the voice or the vibration for assisting the training contents ends.

5. The assist method according to claim 4, wherein the training result includes data configured to indicate a correlation between the function of the automatic nervous system of the user and the function of the automatic nervous system of the dependent or the animal after the training.

6. The assist method according to claim 4 or 5, further comprising a step of providing a notification configured to encourage the user to contact the dependent or the animal before the image, the video image, the voice or the vibration configured to assist the training contents starts.

7. The assist method according to any one of claims 4 to 6, further comprising a step of causing the user to input a questionnaire related to an effect of the training before the start of or after the end of the image, the video image, the voice or the vibration configured to assist the training contents,
wherein the training contents are determined based on at least a result of the questionnaire inputted by the user and the second index.

8. The assist method according to any one of claims 1 to 7, further comprising a step of notifying the user of a timing to start the training.

9. The assist method according to claim 8, wherein the start of the training is notified based on an activity amount of the dependent or the animal in a predetermined period.

10. The assist method according to any one of claims 1 to 9, further comprising a step of calculating a target value of the second index configured to indicate the function of the automatic nervous system of the dependent based on at least one element of a weight at a time of birth, a sex, an age in day and a period from pregnancy to birth of the dependent,
wherein the training contents to be provided to the user are determined based on a difference between the target value and the second index.

11. A program causing the computer to execute the assist method according to any one of claims 1 to 10.

12. An assist system configured to provide a training to the user, the training being configured to assist to enhance a function of an automatic nervous system of a dependent or an animal cared by a user, the assist system comprising:
a sensor configured to monitor an index configured to indicate functions of automatic nervous systems of the user and the dependent or the animal; and
a training contents determination unit configured to provide training contents to the user, the training contents being configured to enhance the function of the automatic nervous system of the user determined based on at least the index configured to indicate the function of the dependent or the animal.
